# FASCICULE DE BREVET EUROPEEN

(11) **EP 1 622 926 B1**
(45) Date de publication et mention de la délivrance du brevet: **08.07.2009**
(21) Numéro de dépôt: 04742578.0
(22) Date de dépôt: 27.04.2004
(51) Int. Cl.: C07J 21/00, C07J 43/00, C07J 75/00

(54) **NOUVEAU PROCEDE ET INTERMEDIAIRES DE PREPARATION DE COMPOSES 19-NOR-STERO DES-17-HALOGENES**
NEUES VERFAHREN UND NEUE ZWISCHENPRODUKTE ZUR HERSTELLUNG VON 17-HALOGEN-19-NORSTEROIDEN
NOVEL METHOD AND INTERMEDIATES FOR THE PREPARATION OF 19-NOR-STEROIDS-17-HALOGEN COMPOUNDS

(30) Priorité: 29.04.2003 FR 0305222
(43) Date de publication de la demande: 08.02.2006
(73) Titulaire: Aventis Pharma S.A., 92160 Antony (FR)
(72) Inventeur: NIQUE, François, F-94170 Le Perreux (FR); MORATILLE, Christian, F-94360 Bry sur Marne (FR); ROUSSEL, Patrick, F-94320 Thiais (FR); BOUSQUET, Joelle, F-69100 Villeurbanne (FR)
(74) Mandataire: Vieillefosse, Jean-Claude
(86) Numéro de dépôt international: PCT/FR2004/001011
(87) Numéro de publication internationale: WO 2004/096829

(56) Documents cités:
- WO-A-99/25725
- WO-A-99/67274
- FR-A- 2 640 977
- US-A- 3 275 623

## Description

La présente invention a pour objet un procédé de préparation de dérivés estrogènes, tels que les 19-nor-stéroides-17-halogénés ainsi que les composés intermédiaires préparés lors de la mise en oeuvre de ce procédé.

L'ostéoporose est une maladie de l'os qui touche 50 millions de personnes dans le monde, plus particulièrement les femmes. Son développement est lié à l'âge et commence le plus souvent après la ménopause.

Cette maladie est caractérisée par une réduction de la densité osseuse, entraîne des déformations, des tassements vertébraux et à terme des fractures spontanées. L'ostéoporose représente donc un sérieux enjeu de santé publique. Le principal traitement consiste dans une prise régulière d'estrogènes qui diminue la perte osseuse, qui cependant peut s'accompagner de certains effets secondaires (saignements, bouffées de chaleur, risque de cancer mammaires...). Une nouvelle série de molécules appelées SERM (Selective Estrogen Receptor Modulator) permet le traitement de l'ostéoporose tout en évitant certains des effets secondaires.

La demande de brevet WO99/67274 décrit de telles molécules de structure 19-nor-stéroïde, halogénées en position 17. Dans cette demande, le procédé de préparation décrit l'halogénation en fin de synthèse.

Cette synthèse a été optimisée :
- en réduisant le nombre d'étapes avec le substrat stéroïde;
- en réalisant l'halogénation dès le début de la synthèse pour rendre la purification plus facile.

L'objet de la présente demande est la mise au point d'un nouveau procédé de préparation d'un intermédiaire clé ou produit fini (composé de formule I) dans la synthèse de certains de ces dérivés estrogènes ayant une activité dissociée.

L'invention a pour objet un procédé de préparation de composés de formule générale (I): 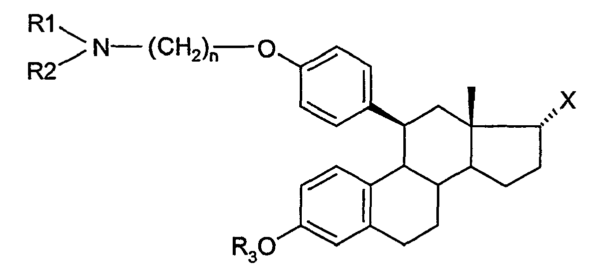 dans laquelle
- soit R₁ et R₂, identiques ou différents représentent un groupement benzyle ou un radical alkyle, alkényle ou alkynyle linéaire, ramifié ou cyclique renfermant de 1 à 8 atomes de carbone
- soit R₁ et R₂ forment ensemble avec l'atome d'azote qui les porte un hétérocycle de 5 à 6 chaînons saturé ou insaturé, aromatique ou non aromatique renfermant éventuellement de 1 à 3 hétéroatomes additionnels et éventuellement accolé à un autre cycle,
X représente un atome d'halogène,
R₃ représente un atome d'hydrogène ou un groupement protecteur de la fonction hydroxy,
n est un entier de 2 à 8,
comprenant les étapes suivantes ;
a) on soumet un composé de formule (II) : 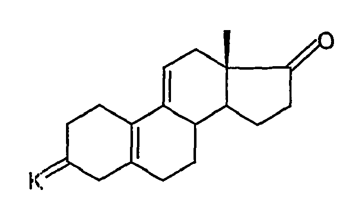 =K représentant une fonction céto protégée notamment sous forme de cétal, de thiocétal ou de cétal mixte,
   à l'action d'un agent réducteur du céto en 17 afin d'obtenir un composé de formule (III) : 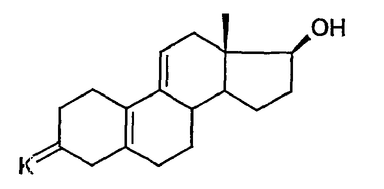
b) on traite le composé de formule (III) avec un agent d'halogénation afin d'obtenir un composé de formule (IV) : 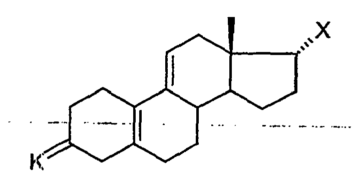 dans laquelle X représente un atome d'halogène,
c) on soumet le composé de formule (IV) à l'action d'un réactif d'époxydation afin d'obtenir le composé de formule (V) : 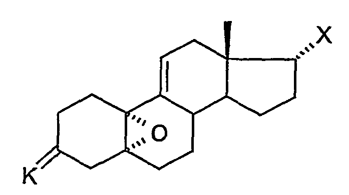
d) on soumet le composé de formule (V) à une réaction d'alkylation avec un dérivé organocuprate dérivé d'un organométallique de formule R₅MgHal ou R₅Li, Hal étant un atome d'halogène et généré de façon catalytique ou stoechiométrique, dans laquelle R₅ représente le groupement : 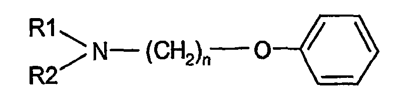 n, R₁ et R₂ étant tels que définis plus haut, la liaison s'effectuant au niveau du phényle,
   puis à l'action d'un agent de déprotection afin d'obtenir le composé de formule (VI): 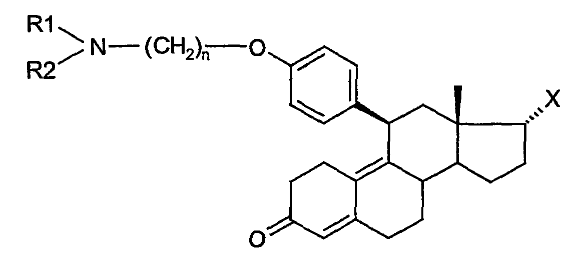
e) on traite le composé de formule (VI) avec un agent d'aromatisation pour obtenir le composé de formule (I),
f) le cas échéant on déprotège le produit obtenu au stade e pour obtenir un composé de formule (I) dans laquelle R₃ représente un atome d'hydrogène, que l'on soumet le cas échéant à une neutralisation et à une salification.

Comme exemple de radical alkyle linéaire ou ramifié renfermant de 1 à 8 atomes carbones on peut citer les radicaux méthyle, éthyle, propyle, butyle, pentyle, hexyle, octyle, et les isomères ramifiés de ces radicaux, isopropyle, isobutyle, isopentyle, neopentyle, isohexyle, 3-méthylpentyle, sec-butyle, tert-butyle, tert-pentyle.

Comme exemple de radical alkyle cyclique on peut citer les radicaux cyclopropyle, cyclobutyle, cyclopentyle et cyclohexyle, cycloheptyle ou cyclooctyle qui peuvent être substitués par exemple par un groupement alkyle renfermant de 1 à 4 atomes de carbone.

Comme exemple de radical alkényle, on peut citer les radicaux allyle, butényle ou 3-méthyl-2-butényle. Comme exemple de radicaux alkynyle on peut citer le radical propargyle. Bien entendu ces radicaux alkényle ou alkynyle renferment au moins 2 atomes de carbone et sont liés à l'atome d'azote via un groupement -CH₂-.

Comme exemple d'hétérocycle que peuvent former R₁ et R₂ ensemble avec l'atome d'azote auquel ils sont liés, on peut citer notamment des hétérocycles mono ou bicycliques renfermant éventuellement un autre hétéroatome choisi parmi l'oxygène et l'azote tels que les hétérocycles insaturés suivants : pyrrolyle, imidazolyle, indolyle, pyridyle, pyrazinyle, pyrimidinyle, pyridazinyle, thiazolyle, oxazolyle, furazolinyle, pyrazolinyle ; ou tels que les hétérocycles saturés suivants : morpholinyle, pyrrolidinyle, piperidinyle, oxazolidinyle, thiazolidinyle.

Il s'agira de préférence du groupement :

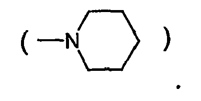

Comme exemple d'atome d'halogène que peut représenter Hal, on peut citer le chlore, l'iode ou le brome. De préférence Hal est un atome de brome.

Comme exemple d'atome d'halogène que peut représenter X, on peut citer le chlore, le brome, l'iode, le fluor. Il s'agit de préférence du fluor.

Comme exemple de groupement protecteur que peut représenter R₃, on peut citer notamment un groupement (C₁-C₆)-alkyle, (C₁-C₆)-alkyle-CO- tel que CH₃CO ou benzoyle, benzyle, phényl-(C₁-C₆)-alkyle tel que benzyle, ainsi que tous les groupements protecteurs connus de l'homme du métier par exemple ceux décrits dans Greene, Wuts, Protective Groups in Organic Synthesis 3rd edition, Wiley & sons, 1999. De préférence R₃, à titre de groupement protecteur, est un groupement acyle.

Comme exemple de groupe protecteur du céto en position 3 du stéroïde que peut représenter =K on peut citer notamment
- les cétals cycliques tels que -O-(CH₂)ₘ-O- , -O-(CH₂)ₘ-S-, -S-(CH₂)ₘ-S-, -O-CH₂-C(C₁₋₄-alkyl)₂-CH₂-O-,
- les cétals acycliques tels que (CH₃O)₂, (EtO)₂
- ainsi que tous les groupements protecteurs du groupement céto connus de l'homme du métier par exemple ceux décrits dans Greene, Wuts, Protective Groups in Organic Synthesis 3rd edition, Wiley & sons, 1999.

L'invention a plus particulièrement pour objet un procédé tel que défini ci-dessus **caractérisé en ce que** =K est un cétal cyclique, et notamment le 3,3-éthylènedioxy.

L'invention a plus particulièrement pour objet un procédé tel que défini ci-dessus **caractérisé en ce que** X représente un atome de fluor.

L'invention a tout particulièrement pour objet un procédé tel que défini plus haut **caractérisé en ce que** R₁ et R₂ forment ensemble avec l'atome d'azote auquel ils sont liés un groupement 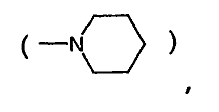 et n est égal à 2.

La réduction du 17-céto en alcool s'effectue selon les méthodes classiques, notamment par action d'un borohydrure alcalin tel que le borohydrure de sodium dans le méthanol ou l'éthanol, ou par action d'hydrure d'aluminium et de lithium dans le THF. Cette réaction permet d'obtenir notamment l'alcool en position 17 bêta. La réduction du 17-céto en alcool est réalisée de préférence par action du borohydrure de sodium dans le méthanol.

La réaction d'halogénation qui suit s'effectue notamment avec des réactifs tels que XSO₂C₄F₉ en présence d'une base encombrée telle que le DBU (diazabicycloundécène), X est de préférence le fluor. D'autres méthodes connues de l'homme du métier peuvent également être utilisées.

La réaction d'halogénation peut notamment s'effectuer en présence de fluorure de perfluorobutane sulfonyle, de complexe acide fluorhydrique/triéthylamine ((HF)₃, TEA) et de DBU.

La réaction d'époxydation est une réaction classique qui s'effectue selon les méthodes connues de l'homme du métier. Elle peut s'effectuer notamment en présence d'hexachloroacétone, de dichlorométhane et d'eau oxygénée.

La réaction d'alkylation avec un dérivé organocuprate dérivé d'un organométallique de formule R₅MgHal ou R₅Li, Hal étant tel que défini plus haut, s'effectue selon les méthodes classiques connues de l'homme du métier.

La réaction de déprotection qui permet d'obtenir le composé de formule (VI) s'effectue selon les méthodes classiques connues de l'homme du métier. L'agent de déprotection utilisé est notamment un agent permettant une hydrolyse acide, tel que l'acide chlorhydrique.

La réaction d'aromatisation suivie de la réaction de saponification s'effectue selon les méthodes classiques telles que décrites dans le brevet européen 0097572. Cette aromatisation peut s'effectuer de préférence en présence de bromure d'acétyle et d'anhydride acétique.

Le cas échéant, la déprotection du groupement acétyle formé s'effectue en général en présence d'une base forte, telle que la soude ou la potasse, dans un alcool, tel que le méthanol ou l'éthanol.

Les réactions de salification et de neutralisation sont effectuées par les méthodes classiques connues de l'homme du métier.

L'invention a également pour objet un procédé de préparation des composés de formule (VI) à partir des composés de formule (II) selon la méthode telle que définie plus haut.

Les composés de formule (II) sont des composés connus ou aisément accessible à l'homme du métier. Notamment les composés de formule (II) dans laquelle =K est un groupement 3,3-éthylènedioxy, sont décrits dans l'article Crocq, V. et al.; Org. Process. Res. Dev. 1997, 1, 2.

L'invention a également pour objet à titre de composés intermédiaires nouveaux,
- le composé de formule générale (IV) dans laquelle =K représente le 3, 3-éthylènedioxy,
- le composé de formule générale (V) dans laquelle =K représente le 3, 3-éthylènedioxy,
- le composé de formule générale (VI) dans laquelle X représente un atome de fluor , n est égal à 2,et R₁ et R₂ forment ensemble avec l'atome d'azote auquel ils sont liés un groupement

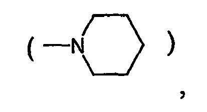

L'invention a plus particulièrement pour objet à titre de composés intermédiaires nouveaux,
- le composé de formule générale (IV) dans laquelle =K représente le 3, 3-éthylènedioxy , n est égal à 2, et X représente un atome de fluor,
- le composé de formule générale (V) dans laquelle =K représente le 3, 3-éthylènedioxy, n est égal à 2, et X représente un atome de fluor.

### Partie expérimentale

### Exemple 1 : 11-bêta-(4-(2-(1-pipéridinyl) éthoxy) phényl)-17-alpha-fluoro-estra-1,3,5(10)-trien-3-ol

### Stade a : Réduction

### 3,3-éthylènedioxy-estra-5(10), 9(11)-dien-17-ol

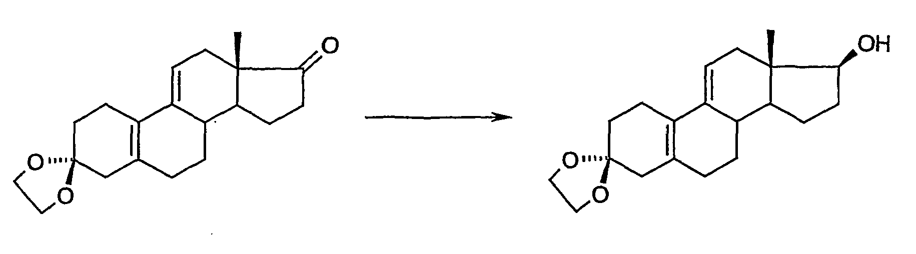

Sur une suspension de 3,3-éthylènedioxy-estra-5(10), 9(11)-dien-17-one (PM = 314.4 ; 100 g ; 318 mmol) dans le méthanol (1 litre) on introduit en 5 mn environ vers 2°C du borohydrure de sodium (PM = 37,8 ; 18.9 g ; 500 mmol) en solution dans de la soude 0.5 N (100 ml). On agite 2 h vers 2°C puis introduit en 15 mn environ vers 5°C de l'acétone (100 ml). On agite 1 h, et coule le milieu vers 20°C dans le mélange agité d'eau (2 litres), de chlorure de sodium (500 g) et d'acétate d'éthyle (400 ml). On décante et ré-extrait la phase aqueuse par de l'acétate d'éthyle. Les phases organiques réunies sont lavées à l'eau, séchées sur sulfate de sodium et concentrées à pression ordinaire à 500 ml. On poursuit la distillation en maintenant le volume constant par introduction progressive de 1,2- diméthoxyéthane (DME). La température en fin d'échange est 83°C. La solution est engagée telle quelle au stade suivant, mais un extrait sec conduit au produit attendu (résine). C₂₀H₂₈O₃ ; PM = 316.4 : IR (CHCl₃, cm⁻¹) : v 3613, 1638.
RMN ¹H (CDCl₃, ppm) : δ 0,74 (s, 3H), 2,29 (sl, 2H), 3,78 (t, J = 8.5 Hz, 1H), 3,98 (m, 4H), 5,57 (m, 1H), de 0.85 à 2,6 (m, 16H).

### Stade b : Fluoration

### 3,3-éthylènedioxy-17-alpha-fluoro-estra-5(10), 9(11)-diène

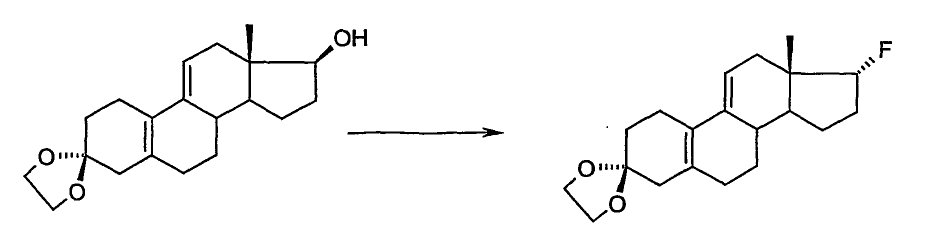

Sur la solution de 3,3-éthylènedioxy-estra-5(10), 9(11)-diène-17-ol (PM = 316.4 ; 20 g ; 63.2 mmol) dans le DME (100 ml) obtenue au stade précédant, on introduit en 5 mn environ vers -10°C du fluorure de perfluorobutanesulfonyle (PM = 302,1 ; 41,4 g ; 137 mmol). On refroidit la suspension vers -40°C et introduit en 30 mn environ à cette température le complexe 3HF.TEA (PM= 161.2 ; 10.2 g ; 63.3 mmol). On agite 15 mn environ vers -40°C puis introduit en une heure environ à cette température la 1,8-diaza-bicyclo(5.4.0)-undéc-7-ène (DBU)(PM= 152.2 ; 38,4 g ; 252 mmol). On agite la suspension jaune 15 mn vers -40°C puis 3 h vers 2°C. On coule le milieu dans le mélange agité d'eau (400 ml), de chlorure d'ammonium (80 g) et d'acétate d'éthyle (140 ml), vers 10°C. On agite 30 mn, décante et ré-extrait avec de l'acétate d'éthyle. On lave les phases organiques réunies par de l'eau et de la soude 1N, sèche sur sulfate de sodium. On concentre sous vide puis introduit du dichlorométhane (300 ml). On introduit sur la solution de la silice (Merck Si60 ; 60 g). On agite une heure vers 20°C, filtre la silice et rince au dichlorométhane (80 ml). On concentre le filtrat à pression ordinaire à 80 ml. On poursuit la distillation en maintenant le volume constant par introduction progressive d'isopropanol. La température en fin d'échange est 82°C. La solution est ramenée vers 20°C en amorce la cristallisation vers 63°C. La suspension est agitée une heure vers 20°C. On essore à 20°C et sèche sous vide une nuit vers 40°C. On obtient 12,34 g de produit blanc (rendement : 61 %) (Tf = 100°C). C₂₀H₂₇O₂F; PM = 318.4.
IR (CHCl₃, cm⁻¹) : v 1640, 1610 ;
RMN ¹H (CDCl₃, ppm) : δ 0,66 (d, J= 2.5 Hz, 3H), 3,99 (sl, 4H), 4,59 (dd, J= 55 et 5 Hz; 1H), 5,60 (m, 1H), de 0,8 à 2,6 (m, 18H);
SM (EI ; m/z) : 318 (M⁺), 298 (M⁺ - HF).

### Stade c : Epoxydation

### 3,3-éthylènedioxy-17-alpha-fluoro-5(10)-époxy-estr-9(11)-ène

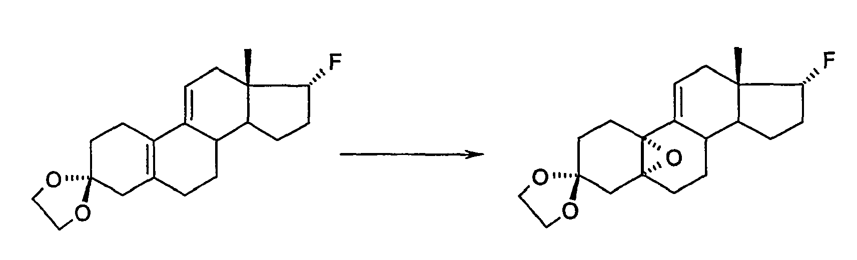

Le 3,3-éthylènedioxy-17-alpha-fluoro-estra-5(10), 9(11)-diène (100 g ; M : 318,4 ; 0,314 mole), l'hexafluoro-acétone (trihydrate ; 22,3 ml ; 0,5 eq.), la pyridine (10 ml), le peroxyde d'hydrogène à 50 % (env. 18 M ; 43,5 ml ; 2,5 eq) et du dichlorométhane (1000 ml) sont agités vigoureusement pendant 18 h à 0-5°C. Après réduction en présence de thiosulfate de sodium aqueux, lavages (eau) et extractions (dichlorométhane), la phase organique est séchée sur sulfate de sodium. On ajoute ensuite de la silice (Merck Si 60, 100 g), agite la suspension 30 mn vers 20°C, filtre et rince au dichlorométhane (200 ml). On concentre le filtrat sous vide à 300 ml, et distille à volume constant par introduction de n-heptane (T de fin d'échange : 98°C). On refroidit sous agitation, l'époxyde alpha cristallise vers 55°C. On agite 16 h vers 20°C, filtre et sèche sous vide vers 40°C. On obtient 53.5 g de solide blanc (rendement : 51 %) (Tf = 115°C). Par chromatographie sur silice des liqueurs mères (éluant : heptane 80- acétate d'éthyle 20), on récupère un second jet de 14 g (soit 13 %); C₂₀H₂₇O₃F ; MW : 334.4 :
IR (CHCl₃, cm⁻¹) : v 1640 ;
RMN ¹H (CDCl₃, ppm) : δ 0,66 (d, J= 2 Hz, 3H), 3,85 à 3,97 (m, 4H), 4,58 (dd, J= 55 et 5 Hz, 1H), 6,07 (dt, J= 5.5 et 2.5 Hz, 1H), de 1,15 à 2,57 (m, 18H).

### Stade d : Alkylation

### 17-alpha-fluoro-11-bêta-(4-(2-(1-pipéridinyl)éthoxy) phényl)-estra-4,9-diene-3-one.

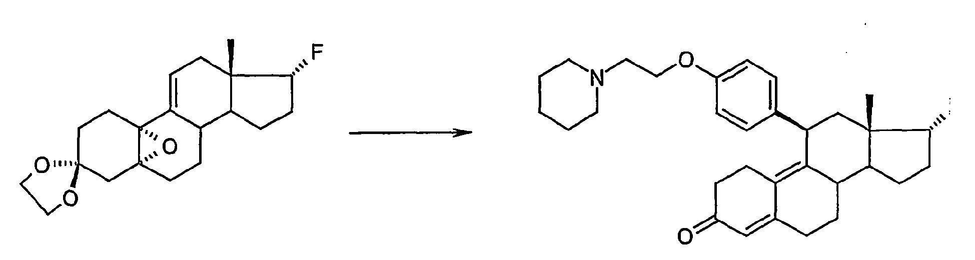

On introduit dans un réacteur 6,7 g de magnésium (tournures ; PM= 24.3 ; 1,8 eq.) puis 56 ml d'une solution de bromure de 4-(2-(1-pipéridinyl)-éthoxy)-benzene (70 g ; PM= 284,2 ; 1,65 eq.) dans le THF (280 ml). On chauffe vers 58°C et dès que le milieu devient gris, on introduit le reste de la solution vers 58°C en 1,5 h environ, puis maintient encore 2 h à cette température. On ramène la solution à 20°C et agite 18 h. On introduit le chlorure cuivreux (1,5 g ; PM= 99.0 ; 0,1 eq.) , agite 5 mn environ vers 20°C puis introduit en 30 mn vers 5°C une solution de 3,3-éthylènedioxy-17-alpha-fluoro-5(10)-alpha-époxy-estr-9(11)-ène (50 g ; PM= 334,4 ; 149 mmol) dans le THF (200 ml). On agite une heure vers -5°C et coule le milieu sur le mélange agité de chlorure d'ammonium (250 g), d'eau (1 litre) et de dichlorométhane (500 ml), vers 10°C. On décante et ré-extrait la phase aqueuse au dichlorométhane. On lave les phases organiques réunies à l'eau, et les concentre sous vide jusqu'à 250 ml environ. On refroidit la solution vers 2°C et introduit de l'eau (125 ml) puis de l'acide chlorhydrique concentré (36% ; 75 ml), toujours vers 2°C. On agite 1.5 h vers 2°C, puis dilue le milieu à l'eau (250 ml), décante et lave à l'eau. On coule en 30 mn environ (formation de mousses) sur le mélange agité vers 20°C de bicarbonate de sodium (23,5 g ; PM= 84.0 ; 1,9 eq.) dans l'eau (250 ml). On agite 30 mn et décante. On ré-extrait les phases aqueuses au dichlorométhane, lave à l'eau et sèche sur sulfate de sodium. On filtre et rince au dichlorométhane. Le filtrat est concentré à pression ordinaire jusqu'à environ 250 ml, puis on continue la distillation en maintenant le volume constant par introduction progressive d'éther isopropylique. La température en fin d'échange est de 68°C. La cristallisation est spontanée. On laisse refroidir vers 20°C et agite encore 2 h vers 20°C. On essore et sèche sous vide vers 40°C. On obtient 56,1 g de solide beige (rendement : 78 %) (Tf = 160°C) : C₃₁H₄₀FNO₂ ; PM : 477.7 ; IR (CHCl₃, cm⁻¹) : 1656, 1608, 1508 ;
RMN ¹H (CDCl₃, ppm) : 0,35 (d, J= 2 Hz, 3H) ; 1,44 (m, 2H) ; 1,60 (m, 4H) ; 2,50 (tl, J= 6 Hz, 4H) ; 2,76 (t, 6 Hz, 2H) ; 4,07 (t, 6 Hz, 2H) ; 4,39 (m, 1H) ; 4,46 (dd, J= 55.5 et 5 Hz, 1H) ; 5,76 (sl, 1H) ; 6,82 et 7,07 (AA'BB', 4H) ; de 1,2 à 4,1 (m, 18H) ;
MS (EI ; m/z) : 477 (M⁺) ; 457 (M⁺-HF) ; 366 ; 346 ; 98.

### Stade e : Aromatisation

### 3-acétyloxy-11-bêta-(4-(2-(1-pipéridinyl)éthoxy) phényl)-17-alpha-fluoro-estra-1,3,5(10)-triène.

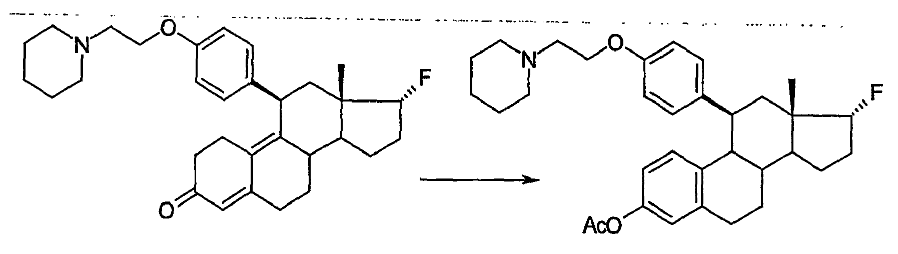

Sur une solution de 17-alpha-fluoro-11-bêta-(4-(2-(1-pipéridinyl)éthoxy)phényl)-estra-4,9-diene-3-one (38 g ; PM= 477,7 ; 79.5 mmol) (stade d) dans le dichlorométhane (152 ml) on ajoute de l'anhydride acétique (PM = 102.1 ; d = 1.09 ; 7.5 ml ; 1.0 eq.) et , en 15 mn, du bromure d'acétyle (PM = 123.0 ; d = 1.66 ; 14.7 ml; 2.5 eq.), à 20-25°C (addition exothermique). La solution marron est agitée pendant 5 h à 20-25°C. On verse la solution en 30 mn environ dans une suspension d'hydrogenocarbonate de sodium (45 g) dans l'eau (380 ml) vers 20°C (dégagement de dioxyde de carbone). Le mélange est agité vigoureusement une nuit vers 20°C, puis la phase organique est décantée, lavée avec de la soude 1 N (190 ml) et de l'eau, puis concentrée jusqu'à un volume final de 114 ml. Le dichlorométhane est remplacé par du méthanol à volume constant par distillation sous vide progressif à environ 40°C. On conserve le produit en solution dans le méthanol. C₃₃H₄₂FNO₃ ; PM : 519.8.

### Stade f : Saponification

### Chlorhydrate de 11-bêta-(4-(2-(1-pipéridinyl)éthoxy) phényl)-17-alpha-fluoro-estra-1,3,5(10)-trien-3-ol

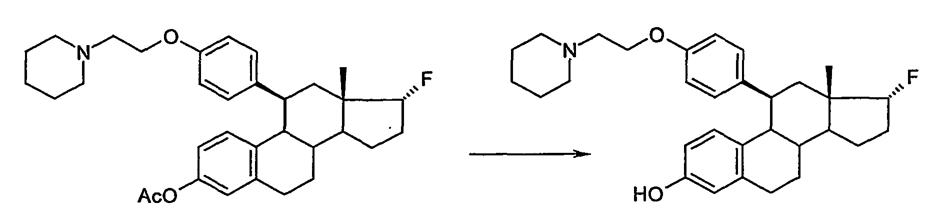

Sur la solution du dérivé fluoré dans le méthanol obtenue au stade e on ajoute en 10 mn environ vers 0°C une solution d'hydroxyde de potassium (PM = 56.0 ; 6.7 g ; 1.5 eq.) dans le méthanol (76 ml). Le milieu est agité pendant 45 mn à 0-5°C, puis versé dans l'eau (190 ml) et le dichlorométhane (190 ml). La phase organique est lavée à l'eau. On acidifie par ajout de méthanol (76 ml), d'eau (190 ml) et d'acide chlorhydrique à 36 % (17 ml ; 2,5 eq.) et agite pendant 5 mn environ en vérifiant le pH (< 2). On décante la phase organique, la sèche sur sulfate de sodium, filtre et concentre jusqu'à obtention d'un volume final de 190 ml. On distille alors à pression ordinaire en maintenant le volume constant par introduction progressive de dichlorométhane (environ 450 ml). Le produit attendu cristallise spontanément. On agite en laissant refroidir en 1 h environ, puis pendant 2 h vers 20°C. Le produit est filtré, lavé avec du dichlorométhane puis séché sous vide vers 40°C. On obtient 30.8 g de solide beige (rendement : 75.3 % ; pureté HPLC : 98 % ) : C₃₁H₄₁ClFNO₂ ; PM : 514.1 ;
IR (CHCl₃, cm⁻¹) : ν = 3599 ; 2467 ; 1609 ; 1583 ; 1511. RMN ¹H (CDCl₃, ppm) : 0,22 (d, J= 1,5 Hz, 3H) ; 3,09 (m, 1H) ; 3,21 (m, 1H) ; 3,87 (m, 1H) ; 3,99 (m, 1H) ; 4,25 (m, 1H) ; 4,43 (dd, J= 56 et 5 Hz, 1H) ; 6,43 et 6,95 (AA'BB', 4H) ; 6,60 (dd, J= 8,5 et 1,5 Hz, 1H) ; 6.67 (d, J= 1,5 Hz, 1H) ; 6,78(d, J= 8,5 Hz, 1H) ; 11,4 (sl, 1H mobile) ; de 0,9 à 3,4 (m, 14H) .
MS (ESP ; m/z) : 478 (MH⁺).

### Stade g : Neutralisation

### 11-bêta-(4-(2-(1-pipéridinyl)éthoxy)phényl)-17-alpha-fluoro-estra-1,3,5(10)-trien-3-ol

Sur une suspension de chlorhydrate de 11-bêta-(4-(2-(1-pipéridinyl)éthoxy)phényl)-17-alpha-fluoro-estra-1,3,5(10)-trien-3-ol (28 g ; PM= 514.1 ; 54.5 mmol) (stade f) dans le dichlorméthane (224 ml), on introduit vers 20°C du carbonate de sodium (PM= 106.0 ; 6.1 g ; 1 eq.) en solution aqueuse (112 ml). On agite 30 mn vers 20°C, décante et lave à l'eau. On sèche la phase organique sur sulfate de sodium, filtre et concentre le filtrat jusqu'à un volume résiduel de 140 ml. On ramène à 20°C et introduit de l'acétone (280 ml), puis de la silice (Merck Si 60 ; 42 g). On agite une heure vers 20°C, filtre et rince avec un mélange acétone-dichlorométhane 2/1.

On concentre le filtrat jusqu'à obtention d'un volume final de 224 ml. On distille alors à pression ordinaire en maintenant le volume constant par introduction progressive d'isopropanol. Le produit cristallise spontanément. On agite en laissant refroidir en 1 h environ, puis pendant 2 h vers 0°C. Le produit est filtré, lavé avec de l'isopropanol vers 0°C puis séché sous vide vers 40°C. On obtient 21.3 g de solide blanc (rendement : 82.1 % ; pureté HPLC : 99 % ; Tf = 180°C) : C₃₁H₄₀FNO₂ ; PM : 477.7 ;
IR (CHCl₃, cm⁻¹) : □ = 3598, 1610, 1581, 1512 ;
RMN ¹H (CDCl₃, ppm) : 0,16 (d, J= 2,5 Hz, 3H) ; 1,34 (m, 2H) ; 1,44 (m, 4H) ; 2,37 (m, 4H) ; 2,56 (t, J= 6 Hz, 2H) ; 3,91 (m, 2H) ; 3,95 (m, 1H) ; 4,44 (dd, J= 56 et 5 Hz, 1H) ; 6,31 (dd, J= 8,5 et 3 Hz, 1H) ; 6.46 (d, J= 3 Hz, 1H) ; 6,63 et 6,97 (AA'BB' , 4H) ; 6,71 (d, J= 8,5 Hz, 1H) ; 8,95 (sl, 1H mobile) ; de 0,9 à 3,0 (m, 13H).

## Revendications

1. Un procédé de préparation de composés de formule générale (I): 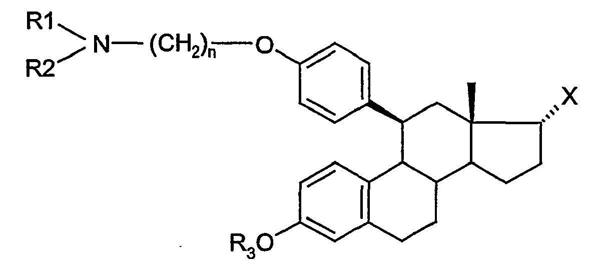 dans laquelle
- soit R₁ et R₂, identiques ou différents représentent un groupement benzyle ou un radical alkyle, alkényle ou alkynyle linéaire, ramifié ou cyclique renfermant de 1 à 8 atomes de carbone
- soit R₁ et R₂ forment ensemble avec l'atome d'azote qui les porte un hétérocycle de 5 à 6 chaînons saturé ou insaturé, aromatique ou non aromatique renfermant éventuellement de 1 à 3 hétéroatomes additionnels et éventuellement accolé à un autre cycle,
X représente un atome d'halogène,
R₃ représente un atome d'hydrogène ou un groupement protecteur de la fonction hydroxy,
n est un entier de 2 à 8,
comprenant les étapes suivantes ;
a) on soumet un composé de formule (II) : 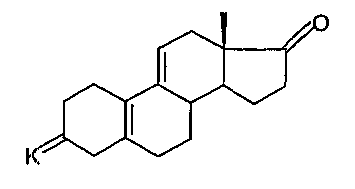 =K représentant une fonction céto protégée notamment sous forme de cétal, de thiocétal ou de cétal mixte,
à l'action d'un agent réducteur du céto en 17 afin d'obtenir un composé de formule (III) : 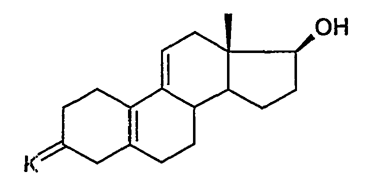
b) on traite le composé de formule (III) avec un agent d'halogénation afin d'obtenir un composé de formule (IV) : 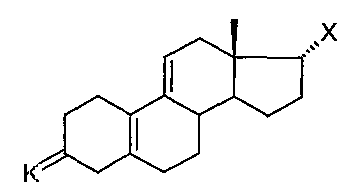 dans laquelle X représente un atome d'halogène,
c) on soumet le composé de formule (IV) à l'action d'un réactif d'époxydation afin d'obtenir le composé de formule (V) : 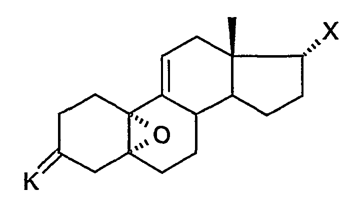
d) on soumet le composé de formule (V) à une réaction d'alkylation avec un dérivé organocuprate dérivé d'un organométallique de formule R₅MgHal ou R₅Li, Hal étant un atome d'halogène et généré de façon catalytique ou stoechiométrique, dans laquelle R₅ représente le groupement : 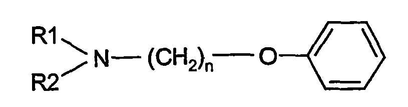 n, R₁ et R₂ étant tels que définis plus haut, la liaison s'effectuant au niveau du phényle,
puis à l'action d'un agent de déprotection afin d'obtenir le composé de formule (VI) : 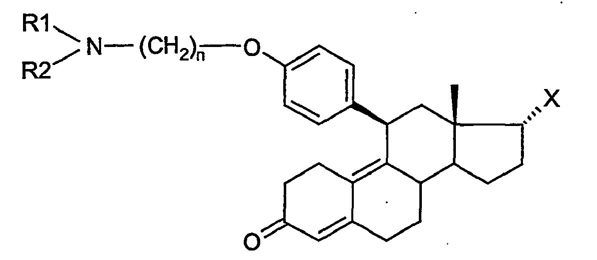
e) on traite le composé de formule (VI) avec un agent d'aromatisation pour obtenir le composé de formule (I),
f) le cas échéant on déprotège le produit obtenu au stade e pour obtenir un composé de formule (I) dans laquelle R₃ représente un atome d'hydrogène, que l'on soumet le cas échéant -à une salification et à une neutralisation.

2. Procédé selon la revendication 1 **caractérisé en ce que** =K représente un cétal cyclique, tel que le 3,3-éthylènedioxy.

3. Procédé selon la revendication 1 ou 2 **caractérisé en ce que** X représente un atome de fluor.

4. Procédé selon la revendication 1, 2 ou 3 **caractérisé en ce que** R₁ et R₂ forment ensemble avec l'atome d'azote auquel ils sont liés un groupement 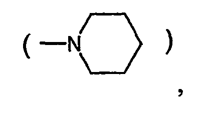 et n est égal à 2.

5. Procédé selon la revendication 1, 2, 3 ou 4 **caractérisé en ce que** la réduction du 17-céto en alcool est réalisée par action du borohydrure de sodium dans le méthanol.

6. Procédé selon la revendication 1, 2, 3 ou 4 **caractérisé en ce que** la réaction d'halogénation est réalisée avec le fluorure de perfluorobutanesulfonyle en présence d'un complexe acide fluorhydique/ triéthylamine et de diazabicyclo-undécène.

7. Procédé selon la revendication 1, 2, 3 ou 4 **caractérisé en ce que** l'agent de déprotection utilisé pour obtenir le composé de formule (VI) est un agent permettant une hydrolyse acide, tel que l'acide chlorhydrique.

8. Procédé selon la revendication 1, 2, 3 ou 4 **caractérisé en ce que** la réaction d'aromatisation s'effectue en présence de bromure d'acétyle et d'anhydride acétique.

9. Procédé de préparation des composés de formule (IV) à partir des composés de formule (II) selon la méthode telle que définie à la revendication 1.

10. A titre de composés intermédiaires nouveaux,
- le composé de formule générale (IV) selon la revendication 1 dans laquelle =K représente le 3, 3-éthylènedioxy,
- le composé de formule générale (V) selon la revendication 1 dans laquelle =K représente le 3, 3-éthylènedioxy,
- le composé de formule générale (VI) selon la revendication 1 dans laquelle X représente un atome de fluor, n est égal à 2, et R₁ et R₂ forment ensemble avec l'atome d'azote auquel ils sont liés un groupement

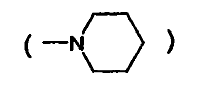

11. A titre de composés intermédiaires nouveaux selon la revendication 10,
- le composé de formule générale (IV) dans laquelle =K représente le 3, 3-éthylènedioxy, n est égal à 2, et X représente un atome de fluor,
- le composé de formule générale (V) dans laquelle =K représente le 3, 3-éthylènedioxy, n est égal à 2, et X représente un atome de fluor.

## Claims

1. A method for preparing compounds of general formula (I): 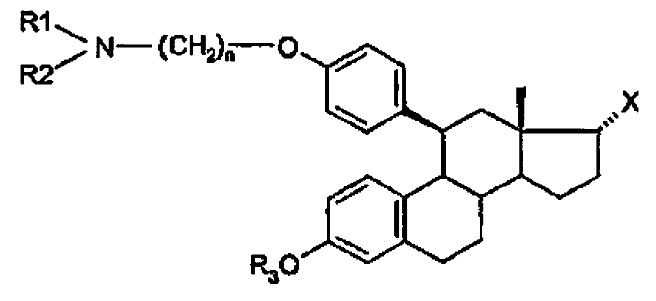 wherein
- either R₁ and R₂, identical or different, represent a benzyl group or a linear, branched or cyclic alkyl, alkenyl or alkylnyl radical containing from 1 to 8 carbon atoms
- or R₁ and R₂ form together with the nitrogen atom which bears them, a saturated or unsaturated, aromatic or non-aromatic heterocycle with 5 to 6 members optionally containing 1 to 3 additional heteroatoms and optionally fused with another cycle,
X represents a halogen atom,
R₃ represents a hydrogen atom or a group protecting the hydroxy function,
n is an integer from 2 to 8,
comprising the following steps;
a) a compound of formula (II): 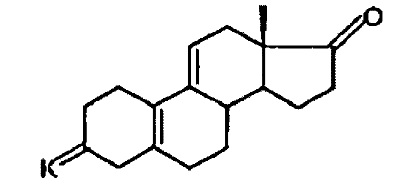 =K representing a protected keto function, particularly as a ketal, thioketal or mixed ketal,
is subjected to the action of an agent for reducing the keto in 17 in order to obtain a compound of formula (III): 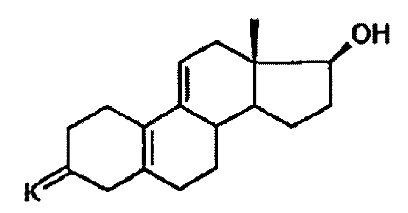
b) the compound of formula (III) is treated with a halogenation agent in order to obtain a compound of formula (IV): 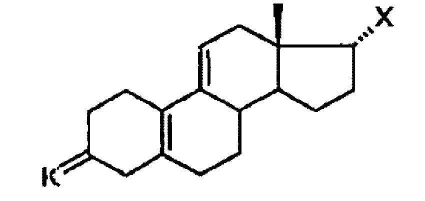 wherein X represents a halogen atom,
c) the compound of formula (IV) is subject to the action of an epoxidation reagent in order to obtain the compound of formula (V): 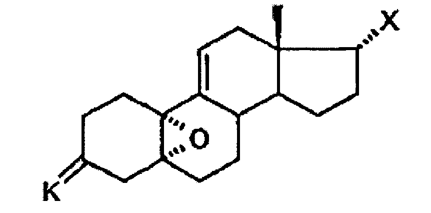
d) the compound of formula (V) is subject to an alkylation reaction with an organocuprate derivative derived from an organometalic compound of formula R₅MgHal or R₅Li, Hal being a halogen atom and generated in a catalytic or stoichiometric way, in which R₅ represents the group: 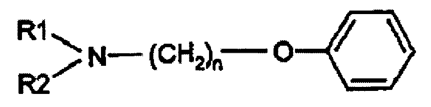 n, R₁ and R₂ being as defined above, the binding being carried out at the phenyl,
and then to the action of a deprotection agent in order to obtain the compound of formula (VI): 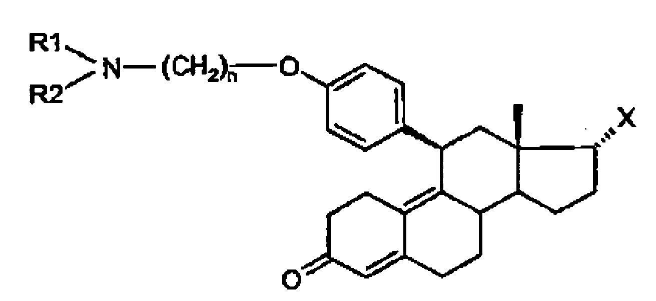
e) the compound of formula (VI) is treated with an aromatization agent in order to obtain the compound of formula (I),
f) if necessary, the obtained product is deprotected in step e) in order to obtain a compound of formula (I) wherein R₃ represent a hydrogen atom, which is optionally submitted to salification and neutralization.

2. The method according to claim 1, **characterized in that** =K represents a cyclic ketal, such as 3,3-ethylenedioxy.

3. The method according to claim 1 or 2, **characterized in that** X represents a fluorine atom.

4. The method according to claim 1, 2 or 3, **characterized in that** R₁ and R₂ form together with the nitrogen atom to which they are bound, a group 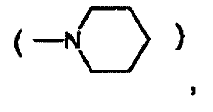 and n is equal to 2.

5. The method according to claim 1, 2, 3 or 4, **characterized in that** reduction of the 17-keto into an alcohol group is achieved by action of sodium borohydride in methanol.

6. The method according to claim 1, 2, 3 or 4, **characterized in that** the halogenation reaction is achieved with perfluorobutanesulfonyl fluoride in the presence of a hydrofluoric acid/triethylamine complex and of diazobicyclo-undecene.

7. The method according to claim 1, 2, 3 or 4, **characterized in that** the deprotection agent used for obtaining the compound of formula (VI) is an agent allowing acid hydrolysis, such as hydrochloric acid.

8. The method according to claim 1, 2, 3 or 4, **characterized in that** the aromatization reaction is carried out in the presence of acetyl bromide and acetic anhydride.

9. A method for preparing compounds of formula (IV) from compounds of formula (II) according to the method as defined in claim 1.

10. As novel intermediate compounds,
- the compound of general formula (IV) according to claim 1 wherein =K represents 3,3-ethylenedioxy,
- the compound of general formula (V) according to claim 1 wherein =K represents 3,3-ethylenedioxy,
- the compound of general formula (VI) according to claim 1 wherein X represents a fluorine atom, n is equal to 2, and R₁ and R₂ form together with the nitrogen atom to which they are bound, a group

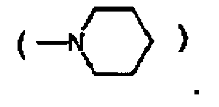

11. As novel intermediate compounds according to claim 10,
- the compound of general formula (IV) wherein =K represents 3,3-ethylenedioxy, n is equal to 2 and X represents a fluorine atom,
- the compound of general formula (V) wherein =K represents 3,3-ethylenedioxy, n is equal to 2 and X represents a fluorine atom.

## Patentansprüche

1. Verfahren zur Herstellung von Verbindungen der allgemeinen Formel (I): 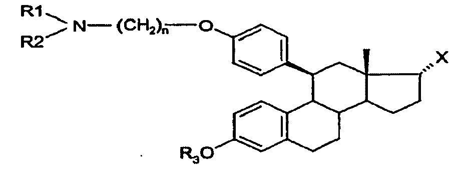 worin
- entweder R₁ und R₂, gleich oder unterschiedlich sind und für eine Benzylgruppe oder einen geradkettigen, verzweigten oder cyclischen Alkyl-, Alkenyl oder Alkynylrest mit 1 bis 8 Kohlenstoffatomen stehen,
- oder R₁ und R₂ gemeinsam mit dem Stickstoffatom, das sie trägt, einen gesättigten oder ungesättigten, aromatischen oder nicht aromatischen 5- bis 6-gliedrigen Heterocyclus mit gegebenenfalls 1 bis 3 zusätzlichen Heteroatomen und gegebenenfalls angefügt an einem anderen Ring bilden,
- X für ein Halogenatom steht,
- R₃ für ein Wasserstoffatom oder eine Schutzgruppe der Hydroxyfunktion steht,
- n eine ganze Zahl von 2 bis 8 ist,
umfassend die folgenden Schritte:
a) das Aussetzen einer Verbindung der Formel (II): 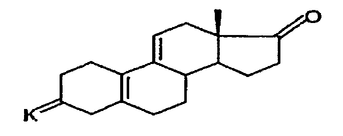 worin =K für eine geschützte Ketofunktion, insbesondere in Form
von Ketal, Thioketal oder gemischtem Ketal, steht,
zu reagieren mit der Wirkung eines 17-Keto-Reduktionsmittels zum Erhalt einer Verbindung der Formel (III): 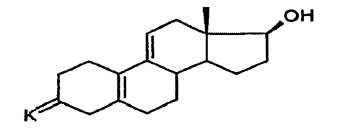
b) das Behandeln der Verbindung der Formel (III) mit einem Halogenierungsmittel zum Erhalt einer Verbindung der Formel (IV): 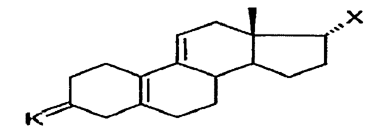 worin X für ein Halogenatom steht;
c) das Aussetzen der Verbindung der Formel (IV) der Wirkung eines Epoxidierungsreagenzes zum Erhalt der Verbindung der Formel (V): 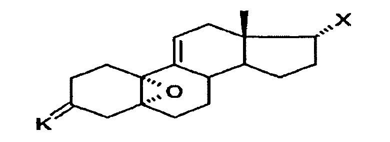
d) das Unterziehen der Verbindung der Formel (V) einer Alkylierungsreaktion mit einem Organocuprat-Derivat, das ein Derivat einer organometallischen Verbindung der Formel R₅MgHal oder R₅Li ist, wobei Hal ein Halogenatom ist, erzeugt auf katalytische oder stöchiometrische Weise, worin R₅ für die folgende Gruppe steht: 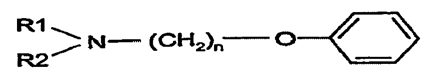 worin n, R₁ und R₂ so wie oben definiert sind, wobei sich die Bindung auf der Ebene des Phenyls vollzieht,
anschließend das Aussetzen einem Mittel zur Schutzgruppenabspaltung zum Erhalt der Verbindung der Formel (VI): 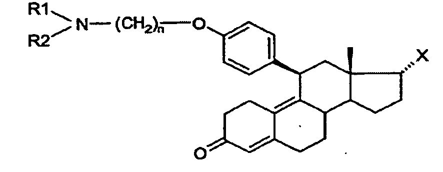
e) das Behandeln der Verbindung der Formel (VI) mit einem Aromatisierungsmittel zum Erhalt der Verbindung der Formel (I),
f) gegebenenfalls das Schutzgruppenabspalten an dem in Schritt e erhaltenen Produkt zum Erhalt einer Verbindung der Formel (I), in der R₃ für ein Wasserstoffatom steht, die gegebenenfalls einer Salzbildung und einer Neutralisierung unterzogen wird.

2. Verfahren nach Anspruch 1, **dadurch gekennzeichnet, dass** =K für ein cyclisches Ketal, wie etwa 3,3-Ethylendioxy, steht.

3. Verfahren nach Anspruch 1 oder 2, **dadurch gekennzeichnet, dass** X für ein Fluoratom steht.

4. Verfahren nach einem der Ansprüche 1 bis 3, **dadurch gekennzeichnet, dass** R₁ und R₂ gemeinsam mit dem Stickstoffatom, an das sie gebunden sind, eine Gruppe 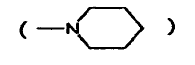 bilden und n gleich 2 ist.

5. Verfahren nach einem der Ansprüche 1 bis 4, **dadurch gekennzeichnet, dass** die 17-Keto-Reduktion zu Alkohol durch die Wirkung von Natriumborhydrid in Methanol erfolgt.

6. Verfahren nach einem der Ansprüche 1 bis 4, **dadurch gekennzeichnet, dass** die Halogenierungsreaktion mit Perfluorbutansulfonylfluorid in Gegenwart eines Fluorwasserstoffsäure/Triethylamin-Komplexes und Diazabicycloundecen erfolgt.

7. Verfahren nach einem der Ansprüche 1 bis 4, **dadurch gekennzeichnet, dass** das Mittel zur Schutzgruppenabspaltung, das verwendet wird, um die Verbindung der Formel (VI) zu erhalten, ein Mittel ist, das eine saure Hydrolyse zulässt, wie etwa Chlorwasserstoffsäure.

8. Verfahren nach einem der Ansprüche 1 bis 4, **dadurch gekennzeichnet, dass** die Aromatisierungsreaktion in Gegenwart von Acetylbromid und Essigsäureanhydrid stattfindet.

9. Verfahren zur Herstellung von Verbindungen der Formel (IV) ausgehend von Verbindungen der Formel (II) gemäß dem in Anspruch 1 definierten Verfahren.

10. Neue Zwischenverbindungen, nämlich
- die Verbindung der allgemeinen Formel (IV) nach Anspruch 1, worin =K für 3,3-Ethylendioxy steht,
- die Verbindung der allgemeinen Formel (V) nach Anspruch 1, worin =K für 3,3-Ethylendioxy steht, und
- die Verbindung der allgemeinen Formel (VI) nach Anspruch 1, worin X für ein Fluoratom steht, n gleich 2 ist und R₁ und R₂ gemeinsam mit dem Stickstoffatom, an das sie gebunden sind, eine Gruppe 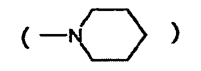
bilden.

11. Neue Zwischenverbindungen nach Anspruch 10, nämlich
- die Verbindung der allgemeinen Formel (IV), worin =K für 3,3-Ethylendioxy steht, n gleich 2 ist und X für ein Fluoratom steht, und
- die Verbindung der allgemeinen Formel (V), worin =K für 3,3-Ethylendioxy steht, n gleich 2 ist und X für ein Fluoratom steht.
